(19) Europäisches Patentamt
European Patent Office
Office européen des brevets

(11) Veröffentlichungsnummer : **0 425 590 B1**

# (12) EUROPÄISCHE PATENTSCHRIFT

(45) Veröffentlichungstag der Patentschrift : **15.12.93 Patentblatt 93/50**

(21) Anmeldenummer : **90901532.3**

(22) Anmeldetag : **18.01.90**

(86) Internationale Anmeldenummer : **PCT/CH90/00012**

(87) Internationale Veröffentlichungsnummer : **WO 90/11348 04.10.90 Gazette 90/23**

(51) Int. Cl.$^5$ : **C12N 5/00,** A01G 31/00, A01K 63/04, C02F 1/66, A61L 15/24

## (54) EIN VERFAHREN ZUR STABILISIERUNG DES pH-WERTES IN WÄSSRIGEN NÄHRLÖSUNGEN SOWIE ZUR ABSORPTION UND PUFFERUNG VON URIN UND WUNDSEKRETEN.

(30) Priorität : **29.03.89 CH 1127/89**

(43) Veröffentlichungstag der Anmeldung : **08.05.91 Patentblatt 91/19**

(45) Bekanntmachung des Hinweises auf die Patenterteilung : **15.12.93 Patentblatt 93/50**

(84) Benannte Vertragsstaaten : **BE DE DK ES FR GB IT NL SE**

(56) Entgegenhaltungen :
**GB-A- 2 185 998**
**US-A- 3 770 603**
**US-A- 4 615 978**
**Anal. Chem., vol. 61, 1989, Pier Giorgo Righetti et al., p. 1602-1612**

(73) Patentinhaber : **MARIGEN S.A.**
**Eugster, Carl, Hackbergstrasse 40**
**CH-4125 Riehen (CH)**

(72) Erfinder : **EUGSTER, Carl**
**Hackbergstrasse 40**
**CH-4125 Riehen (CH)**
Erfinder : **RIGHETTI, Pier, Giorgio**
**Via Archimede, 114**
**I-20129 Milano (IT)**

(74) Vertreter : **Haldemann, Walter, Dr.**
**c/o MARIGEN S.A. 40, Hackbergstrasse**
**CH-4125 Riehen (CH)**

EP 0 425 590 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Landpflanzen, pflanzliche und tierische Zellen können in einer wässrigen Lösung (als hydroponische Kultur) angezogen werden, welche alle essentiellen anorganischen Ionen in geringer Konzentration enthält und ausreichend belüftet wird.

Die Liste der allgemein essentiellen Nährstoffe umfasst 16 Elemente, welche in sehr unterschiedlicher Menge von der Pflanze benötigt werden.

Zu den Elementen, die in erheblichen und leicht messbaren Mengen erforderlich sind, gehören neben C, H und O auch N, K, Ca, Mg und S (Makroelemente).

Für eine vollständige Entwicklung der Pflanzen und Zellen, bzw. Zell-Linien sind eine Anzahl weiterer Elemente unentbehrlich, welche nur in verhältnismässig kleiner Menge angeboten werden müssen (die Mikroelemente), wie Cl, B, Fe, Mn, Zn, Cu und Mo. Darüber hinaus treten bei manchen Pflanzen noch zusätzliche Bedürfnisse auf, z.B. für $SiO_2$ als Gerüstsubstanz bei Gräsern oder Schachtelhalmen oder für Co bei allen Pflanzen, welche auf eine Symbiose mit $N_2$-fixierenden Bakterien angewiesen sind. Auch Na und Se müssen in speziellen Fällen als Mikroelemente angesehen werden.

Bei einer wässrigen Lösung kommt es nicht nur auf die Vollständigkeit hinsichtlich aller benötigten Ionen an, sondern vor allem auch auf die Gesamt-Ionen-Stärke, den pH-Wert, die Pufferkapazität und das Verhältnis zwischen den einzelnen Ionen.

Von besonderer Bedeutung ist der pH-Wert, vorab im Hinblick auf die Verfügbarkeit der angebotenen Nährstoffe (Makro- und Mikroelemente). Dies beruht auf ihrer unterschiedlichen Löslichkeit in Abhängigkeit vom pH-Wert. Im Bereich niedriger pH-Werte (ca. 3 - 4,5) ist mit Ausnahme von Eisen und Mangan durchwegs eine geringe Verfügbarkeit von Nährstoffen gegeben.

Im Bereich mittlerer pH-Werte (ca. 5 - 7) kann meist eine hohe Verfügbarkeit der Pflanzennährstoffe nachgewiesen werden, während im Bereich höherer pH-Werte (ca. 7 - 8,5) mit Ausnahme des Calziums, Magnesiums und Molybdäns die Verfügbarkeit wieder abnimmt. Auch die Nitrifizierung, die als biologischer Vorgang auf der Tätigkeit von Mikroorganismen beruht, ist pH-abhängig. Bei Werten unterhalb von 6 werden die Umsetzungsintensitäten der beteiligten Organismen eingeschränkt. Im Bereich hoher pH-Werte (über 8) verlaufen Nitrierungsprozesse verlangsamt.

Pflanzen, die an saure Verhältnisse adaptiert sind, d.h. an pH-Werte unter 5,5 , finden sich im wesentlichen nur unter Wildpflanzen. Die pH-Ansprüche der meisten Kulturpflanzen liegen im Bereich von 5,5 - 7 . Die Aufnahme von Anionen wird im Bereich hoher pH-Werte vermindert, jene der Kationen im Bereich niederer pH-Werte.

Der Einfluss kann sehr erheblich sein, so dass eine Verschiebung des pH-Wertes um 1 - 2 Stufen bereits Aenderungen in der Aufnahme bis zu 50% bewirken kann.

Aus dem US-Patent 4'152'215 ist eine apparative Methode bekannt, welche auf der Basis der Elektrolyse durch eine Ionenaustausch-Membran den pH-Wert in hydroponischen Lösungen kontrolliert und stabilisiert.

Ueberraschenderweise wurde nun ein Verfahren gefunden, womit sich unter Zugabe geeigneter synthetischer Trägerampholyte eine hohe Pufferkapazität, sowie eine dauerhafte pH-Steuerung in hydroponischen Lösungen erzeugen lassen. Dies ist möglich ohne zusätzlichen apparativen Aufwand.

Die erfindungsgemäss zu verwendenden synthetischen Trägerampholyte haben geladene, in einer Matrix unbeweglich gemachte Gruppen, welche eines oder mehrere der Elemente Kohlenstoff, Schwefel, Phosphor, Bor oder Stickstoff enthalten.

Diese nicht-beweglichen, geladenen und/oder aufladbaren Gruppen der Trägerampholyte können durch Abspaltungs-bzw. Protolysengleichgewichte inbezug auf die verwendete Nährlösung, positive oder negative Ladungen abgeben.

Beispiele negativ geladener Gruppen sind Carboxyl-, Schwefelsäure-, Borsäure-, oder Phosphorsäuregruppen. Dazu zählen auch Säureester und mehrwertige organische Säuregruppen. Positiv geladene Gruppen können z.B. verschiedene Arten von Aminogruppen sein. Die Unbeweglichkeit dieser Gruppen lässt sich dadurch erzielen, dass sie auf oder in eine Matrix, welche z.B. aus einem Gel bestehen kann, gesetzt werden.

Das Unbeweglich-machen bedeutet, dass kein Transport der geladenen Gruppen in der Nährlösung erfolgt. Die Schwingungen, welche die Gruppen wegen der Flexibilität der erfindungsgemäss zu verwendenden Matrixstruktur aufweisen, beeinträchtigen das vorbeschriebene Prinzip nicht.

Um in der Nährlösung ein optimales Pufferleistungsvermögen zu erlangen, sollten die nicht-beweglichen geladenen Gruppen in einem bestimmten Abschnitt der Matrix pK-Werte besitzen, die nahe dem pH-Wert des Matrix-Abschnittes sind. Der beste Richtwert für die Konzentration der geladenen Gruppe wird durch die praktischen Gegebenheiten vorbestimmt. In den meisten Fällen reicht eine Menge von $10^{-1}$ bis $10^{-6}$, vorzugsweise von $10^{-2}$ bis $10^{-4}$ M pro Liter Nährlösung geladene Gruppen aus, um die gewünschte Pufferkapazität, bzw. die erforderliche pH-Stabilität zu erzielen.

Die nach Massgabe der Erfindung einzusetzende Matrix kann ein bekanntes, die Konvektion stabilisierendes System sein, das z.B. netzförmig ("macroreticulated"), gekörnt oder faserig ausgebildet ist.

Als Matrix eignen sich die folgenden Stoffe: Polyacrylamid, Zellulose, Agarose, Dextran, Polyvinylalkohol, Stärke, Siliziumgel oder Polymere des Styrols oder auch Kombinationen derselben.

Zum Einbau der geladenen Gruppen in die Matrix können verschiedene Verfahren angewandt werden. So lassen sich geeignete Derivate von Polyacrylamid durch Copolymerisation herstellen. Fast alle konjugierten, polaren Vinylmonomeren bilden mit Acrylamid geeignete Copolymerisate. Auf diese Weise erhält man schon eine grosse Auswahl an Möglichkeiten für den Einbau von geladenen, nicht-beweglichen Gruppen in die Matrix.

Beispiele von funktionellen Monomeren, welche durch Reaktion zu einem Acrylamid enthaltenden Gel gebracht werden können, sind: Vinylsulfat, Acrylsäure, Vinylphosphorsäure, Maleinsäure, Fumarsäure, Itakonsäure, Aconitsäure, Vinylpyridin, Allylamin und Diallylamin. Alle funktionalen Derivate dieser Verbindungen, z.B. Ester der Säuren wie Aminoalkylacrylat, lassen sich auch mit Acrylamid copolymerisieren.

Einige der vorstehend erwähnten Monomeren erzeugen bereits bei der Verwendung von Bisacrylamid oder anderen Vernetzungsmitteln Gele, ohne dass Acrylamid als Komponente im Polymer enthalten ist. Um die gewünschte Pufferkapazität zu erhalten, kann die Polymerisation auf herkömmlichem Wege in einer Wasserlösung durchgeführt werden. Zum Start der Polymerisation werden Peroxyde, Azoverbindungen, Redoxsysteme, photochemische Systeme und/oder Ionenbestrahlung eingesetzt.

Die Chemie der Puffersysteme mit unlöslichen, ionisierbaren Gruppen wird in der Literatur schon eingehend beschrieben. (Vgl. z.G. J.X. Khym, Analytical Ion-Exchange Procedures in Chemistry and Biology, Prentice Hall, Englewood Cliffs, N.Y., 1974).

Diese Puffersysteme können in Form von porösen Kügelchen (Granulatform, sog. "auto buffered beads"), in fasriger Form oder in anderen Packungsformen hergestellt und eingesetzt werden. Ueberdies lassen sich solche Puffersysteme auf poröses oder nicht-poröses Glas, auf Dialysebeutel, Zellulose, Papier oder auf jede Art/Typ von Plastikmaterial (wie z.B. Nylon, Polypropylen, Polystyrol, usf.) oder auf andere poröse wie auch nicht-poröse anorganische oder organische Oberflächen auftragen. Die Puffersysteme sind vorzugsweise Copolymere vom Polyacrylamid-Typ; sie werden durch Copolymerisation von neutralen, die inerte Matrix bildenden Monomeren wie z.B. Acrylamid oder N,N'-Methylen-Bisacrylamid (vernetzendes Monomer) mit Monomeren von schwachen Acrylamidsäuren oder -basen der allgemeinen Formel

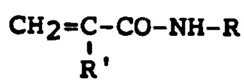

hergestellt. Dabei stellt in dieser Formel R z.B. eine Carboxyl- oder eine primäre, sekundäre oder tertiäre Aminogruppe und R' Wasserstoff oder eine $C_1$ - $C_4$ Alkylgruppe dar. Unter $C_1$ bis $C_4$ Alkyl ist Methyl, Aethyl, Propyl, Isopropyl, n-, i-, sek. oder tert.-Butyl zu verstehen.

Beispiele von besonders bevorzugten Aminogruppen bei R sind u.a.:

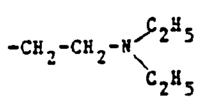

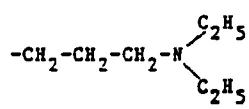

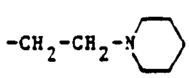

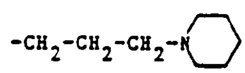

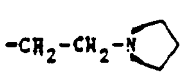

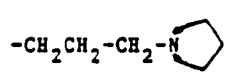

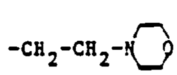

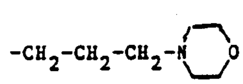

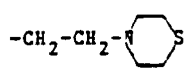

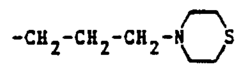

Je nach dem Verhältnis der für die Copolymerisation eingesetzten neutralen, schwach sauren oder basischen Monomeren haben die resultierenden Polymerisate einen bestimmten isoelektrischen Punkt (pI-Wert). Das protolytische Gleichgewicht dieser Polymerisate kann man mit der klassischen Henderson-Hasselbach Gleichung (H-H) berechnen:

$$pH = pK_i + \log [(B_i) / (A_i)]$$

Darin stellt $pK_i$ die Dissoziations-Konstante von irgendeinem der eingesetzten schwachsauren, bzw. basischen Monomeren, $(A_i)$ die molare Konzentration des Acrylamido-Monomeren in seiner sauren Form, bzw. (Bi) die korrespondierende Molarität des Acrylamid-Monomeren in seiner basischen Form dar.

Die H-H Gleichung stellt die Abhängigkeit des pH-Wertes von der totalen Konzentration der für die Copolymerisation eingesetzen Acrylamidosäuren und -Basen dar. Setzt man für die Copolymerisation nur zwei dieser schwach sauren, bzw. basischen Monomere ein, von denen eines vollständig ionisiert ist, so kann man den pH-Wert direkt aus der Acrylamido-Pufferkonzentration berechnen. Dies geschieht mithilfe der folgenden, leicht modifizierten H-H Gleichungen, in Abhängigkeit davon, ob die Puffergruppen im Copolymeren sauer oder basisch sind:

$$pH = pK_A + \log [C_B / (C_A - C_B)]$$

wenn die Puffergruppe im Copolymerisat z.B. eine Carboxylgruppe (eine azifizierende Gruppe) ist oder

$$pH = pK_B + \log [(C_B - C_A) / C_A]$$

wenn die Puffergruppe im Copolymerisat z.B. eine primäre, sekundäre oder tertiäre Aminogruppe (eine basifizierende Gruppe) darstellt.

In den Formeln sind $C_A$ die Molarität des sauren Monomeren mit einem pK-Wert = $pK_A$ und $C_B$ die Molarität des basischen Monomeren mit einem pK-Wert = $pK_B$.

Durch den Einsatz geeigneter, schwach saurer, bzw. basischer Acrylamido-Monomeren kann man so puffernde Copolymerisate herstellen, mit definierten pK-Werten innerhalb des pH-Wert-Intervalles von 3 bis 10 , mit einem präzisen isoelektrischen Punkt und jeder gewünschten Pufferkapazität. (Vgl. dazu z.B. P.G. Righetti, 1983: Isoelectric Focusing. Theory, Methodology and Applications. Elsevier, Amsterdam, Seiten 154-165 oder P.G. Righetti, 1984: Journal of Chromatography 300, Seiten 165-223).

Die vorliegende Erfindung betrifft auch ein Mittel, sowie die Verwendung dieser puffernden Copolymerisate zur Stabilisierung des pH-Wertes in wässrigen Lösungen für hydroponische Landpflanzen-, für Wassertier-, oder Zell-Kulturen und zur Absorption von Urin und Wundsekreten. Solche Anwendungsformen enthalten einen synthetischen Trägerampholyten, welcher auf poröses oder nicht-poröses Glas, auf Dialysebeutel, Zelluloselagen von Windeln, steriles Verbandmaterial oder auf jeden Typ von Plastikmaterial (wie z.B. Nylon, Polypropylen, usw.) aufgetragen ist. Sie liegen in Form von porösen Kügelchen (Granulatform, "auto buffered beads Mariperl®"), in fasriger Form oder in anderen Packungsformen vor. Bevorzugt sind Mittel, bei denen der Trägerampholyt aus einem Copolymeren vom Polyacrylamid-Typ, wie vorbeschrieben, besteht.

N.B. MARIPERL® ist eine eingetragene Marke (reg. ™) der Firma **MARIGEN S.A., RIEHEN.**

**Beispiele zur Herstellung von erfindungsgemäss geeigneten Puffersystemen mit definiertem, isoelektrischem Punkt (pI) und mit hoher Pufferkapazität (β)**

Beispiel 1 :

**Puffersystem mit einem pI = pH-Wert von 5,6**

100 mM monomeres 2-Morpholino-Aethylacrylamid mit einem pK-Wert von 6,2 und 89 mM monomere 4-Acrylamido-Buttersäure mit einem pK-Wert von 4,6 werden in einer wässrigen Lösung von 10 Volumenteilen Acrylamid (neutrales Monomer) und 8 Volumenteilen N,N'-Methylen-bis-Acrylamid (vernetzendes, neutrales Monomer) während einer Stunde bei 50° C in Gegenwart eines Katalyten, wie z.B. TEMED und Ammoniumpersulfat, copolymerisiert.

Vor der Einleitung der Polymerisation mit dem Katalyten wird die Monomeren-Lösung entgast (Entfernung des die Polymerisation störenden Sauerstoffes) und unter kräftigem Rühren in Leichtparaffinöl (3-faches Volumen der Monomerlösung) eingegossen. Es entsteht ein polydisperses Granulat mit einem Teilchendurchmesser zwischen 100 und 500 µm. Aus dem Granulat wäscht man nach dem Abnutschen den Katalysator mehrmals mit Wasser aus. Wie die Analyse zeigt, sind 90 % der eingesetzten Monomeren im Copolymerisat aufgenommen worden.

Dieses amphotere Copolymerisat hat einen pI = pH-Wert von 5,6 (berechnet aus der H-H-Gleichung durch Einsetzen der entsprechenden pK-Werte des 2-Morpholino-Aethylacrylamides und der 4-Acrylamidobuttersäure) und eine totale Pufferkapazität β von 53 Milliaequivalent / . $L^{-1}$ . $pH^{-1}$ (berechnet als Summe der Teil-β-Werte der beiden ionisierbaren Verbindungen. Beide Acrylamid-Abkömmlinge wirken gleichzeitig als Puffer und Titriersubstanz.

Beispiel 2 :

**Puffersystem mit einem pI = pH-Wert von 7,2.**

120 mM monomeres 3-Morpholino-Propylacrylamid mit einem pK-Wert von 7 und 40 mM 4-Acrylamido-Buttersäure mit einem pK-Wert von 4,6 werden in einer wässrigen Lösung von 10 Volumenteilen Acrylamid (vernetzendes, neutrales Monomer) wie im Beispiel 1 bei 50° C während einer Stunde in Gegenwart eines Katalyten copolymerisiert. Bei dieser Polymerisation kann die 4-Acrylamido-Buttersäure z.B. durch N-Acryloyl-Glyzin (pK = 3,6) und 2-Acrylamido-2-Methylpropansulfonsäure (pK = 1) ersetzt werden. Dieses amphotere Copolymerisat hat einen pI = pH-Wert von 7,2 , berechnet aus der H-H Gleichung durch Einsetzen der entsprechenden pK-Werte des 3-Morpholino-Propylacrylamides und der Acrylamidobuttersäure sowie eine totale Pufferkapazität β von 62 MilliAequivalent . $L^{-1}$ . $pH^{-1}$.

Die **Anwendung** der in den Beispielen 1 und 2 hergestellten amphoteren Copolymerisate zur Pufferung von wässrigen Nährlösungen für Landpflanzen-, Wassertier- und Zell-Kulturen:

Beispiel 3 :

Die Pufferung einer hydroponischen Lösung für **Endivienkulturen während der Aufzucht.**

Es ist bekannt, dass Endivienkulturen in einer leichtsauren hydroponischen Lösung (pH 5,6 oder sogar 6,2) optimal aufwachsen. (Forçage). Für den Wachstumsversuch zieht man zuerst in feuchtem Sand Keimlinge von Endivienpflanzen bis zum Zweiblattstadium an. Die Wurzeln von je 5 Keimlingen werden dann durch die Löcher eines Plastikhalters in je 100 ml folgender Nährlösung getaucht:

TABELLE 1 (Nitrat-Vrb.)

| Makroelemente | Mengen (g/l) |
|---|---|
| $KNO_3$ | 0,505 |
| $Ca(NO_3)_2 \cdot 4 H_2O$ | 1,18 |
| $Mg SO_4 \cdot 7 H_2O$ | 0,49 |
| $KH_2PO_4$ | 0,14 |
| **Mikroelemente** | **Mengen (mg/l)** |
| $Mn SO_4 \cdot H_2O$ | 0,77 |
| $Cu SO_4 \cdot 5 H_2O$ | 0,08 |
| $(NH_4) Mo SO_4 \cdot 4 H_2O$ | 0,46 |
| $H_3BO_3$ | 1,46 |
| Fe-EDTA (Sequestren CIBA-GEIGY) | 3,37 |

24 Stunden vor dem Ansetzen der Endivienkeimlinge werden 10 Volumenprozente, bezogen auf das Volumen der Nährlösung, Granulat des im Beispiel 1 hergestellten Copolymerisates mit einem pI = pH-Wert von 5,6 zugefügt. Zur Kontrolle werden je fünf Endivienkeimlinge in 100 ml Nährlösung aufgezogen, bei welcher der pH-Wert der Nährlösung während der Wachstumsperiode einmal nicht reguliert und zum andern Mal nur alle 24 Stunden frisch wieder auf pH 5,6 eingestellt wird. Die Kulturgefässe werden während der Wachstumsperiode bei 22° C und 75% relativer Luftfeuchtigkeit gehalten und die Nährlösungen durch Einpumpen komprimierter Luft gerührt.

Auswertung der Versuchsergebnisse:
Vergleiche die Figur 1 in der technischen Beilage. Wie diese Graphik zeigt, wird in der Nährlösung während der Wachstumsperiode von 14 Tagen der pH-Wert durch den Einsatz des nach Massgabe der Erfindung verwendeten amphoteren Copolymerisates (Beispiel 1) ziemlich konstant bei 5,6 bis 6 gehalten.

Im Gegensatz dazu steigt der pH-Wert in der Kontroll-Lösung ohne Pufferzusatz von 5,6 auf 7,5 (das Wachstum der Pflanzen ist bei diesem pH-Wert schon stark gestört). Ebenso ist ein merklicher pH-Wert Anstieg feststellbar in der Kontrollnährlösung, in welcher man den pH-Wert alle 24 Stunden wieder auf 5,6 zurücktitriert hat. Die pH-Wert Messungen werden dabei alle 24 Stunden vor dem Zurücktitrieren auf 5,6 durchgeführt.

Am Ende der Wachstumsperiode von 14 Tagen werden die Längen der feuchten Wurzeln der Endivienpflanzen gemessen. Die Wurzellänge jener Endivienpflanzen, welche in Nährlösungen mit amphoterem Copolymerisatpuffer wie im Beispiel 1 aufgezogen worden sind, beträgt dabei 26 bis 31 cm, während die Wurzellänge der Pflanzen aus den Kontroll-Nährlösungen 15 bis 20 cm misst.

Beispiel 4 :

Bei diesem Versuch werden die Endivienkeimlinge in Nährlösungen analog dem Beispiel 3 aufgezogen, welche ebenfalls die in der Tabelle 1 angegebenen Mikroelemente, jedoch folgende Makroelemente enthalten:

TABELLE 2 (Ammonium-Vrb.)

| Makroelemente | Konzentration (g/l) |
| --- | --- |
| $(NH_4)_2SO_4$ | 0,62 |
| $Ca\ (H_2PO_4)_2 \cdot H_2O$ | 0,24 |
| $K_2SO_4$ | 0,60 |
| $Ca\ SO_4 \cdot 2\ H_2O$ | 0,47 |
| $Mg\ SO_4 \cdot 7\ H_2O$ | 0,49 |

Wie die Graphik der Figur 2 zeigt, wird der pH-Wert der Nährlösung durch den Einsatz des erfindungskonform zu verwendenden amphoteren Copolymerisates (Beispiel 1 : pH = pI-Wert von 5,6) während der Wachstumsperiode von 14 Tagen konstant bei 5,6 bis 5,0 gehalten.
Im Gegensatz dazu sinkt der pH-Wert in der Lösung ohne Pufferzusatz von 5,6 auf 3, und in der Lösung, in welcher man den pH-Wert alle 24 Stunden wieder auf 5,6 zurücktitriert hat, sinkt er von 5,6 auf 4.

Die biologischen Daten werden in der Graphik der Figur 3 in der technischen Beilage dargestellt. Wie dargetan, haben sich die Endivienpflanzen, welche in der übereinstimmend mit der Erfindung gepufferten Nährlösung aufwuchsen, während der Wachstumsperiode von 14 Tagen deutlich besser entwickelt, als diejenigen aus den beiden Kontroll-Lösungen.

Bei den getrockneten Wurzeln dieser Pflanzen ist dabei gegenüber jenen aus den nicht gepufferten Medien eine dreifache Gewichtszunahme feststellbar. Dies verdeutlicht, dass die Gewichtszunahme bei den feuchten Blättern und Wurzeln nicht auf eine mögliche, verstärkte Wasserquellung durch das zugesetzte amphotere Copolymerisat (Beispiel 1) zurückzuführen ist, sondern allein auf einer echten Wachstumszunahme des pflanzlichen Gewebes beruht.

Beispiel 5 :

**Erfindungsgemässe Pufferung von wässrigen Kulturmedien für Zellen und Zell-Linien während der Wachstumsperiode.**

Es ist bekannt, dass pflanzliche und tierische Zellen in den ersten Tagen ihres Wachstums sehr empfindlich auf pH-Wechsel im wässrigen Kulturmedium reagieren. Um eine Zell-Lysierung zu vermeiden, muss daher das wässrige Kulturmedium optimal während der ganzen Wachstums-tumsperiode bei einem pH-Wert von 7,2 gehalten werden.

Im allgemeinen wird der pH-Wert von 7,2 des wässrigen Kulturmediums während des Wachstums der Zellen durch den kombinierten Einsatz eines Phosphatpuffers und die Einleitung von $CO_2$-Gas aufrechterhalten, wobei übrigens das Gleichgewicht $H_3CO_2 \rightleftarrows HCO_3^- + H^+$ einen pK-Wert von 6,3 hat. Die Pufferkapazität dieses kombinierten Einsatzes genügt aber nicht, um die während des Wachstums im Nährmedium entstehenden

sauren Substanzen bei einem pH-Wert von 7,2 zu stabilisieren.

Im Einklang mit der Erfindung wurde demnach angestrebt, das wässrige Kulturmedium durch den Zusatz des gemäss Beispiel 2 hergestellten amphoteren Copolymerisates, mit einem pI = pH-Wert von 7,2 konstant zu halten. Hiefür wird das amphotere Copolymerisat mit einem pI = pH-Wert von 7,2 vor dem Einsatz in das Kulturmedium im Autoklaven sterilisiert und während 24 Stunden im Nährmedium (10%-Kalbserum-Lösung) vorkonditioniert.

In 500 ml von diesem durch Ultrafiltration sterilisierten Nährmedium werden pro ml zwei Mal 1o$^5$ Hybridomazellen von Säugetieren ohne $CO_2$-Zufuhr während 6 Tagen gehalten. Die Anzahl der Zellen und der pH-Wert werden alle 24 Stunden kontrolliert.

Wie die obere Graphik der Figur 4 ausweist, wird der pH-Wert des Kulturmediums durch den Einsatz von 10 Volumenprozenten amphoterem Copolymerisat (hergestellt gemäss Beispiel 2, mit einem pH-Wert von 7,2) während der Wachstumsperiode auch trotz der Produktion von Milchsäure durch die Zellen konstant zwischen einem pH-Wert von 7,1 und 7,2 gehalten.

Im Gegensatz dazu fällt der pH-Wert des nicht gepufferten Nährmediums auf 6,7.

Gemäss der unteren Graphik der Figur 4 steigt die Wachstumskurve beider Zellpopulationen anfänglich vergleichbar stetig an. Gegen Ende der Wachstumsperiode nimmt jedoch im ungepufferten Medium die Zahl der vollständig lysierten Zellen stark zu.

Wird das amphotere Copolymerisat in biotechnischen Medien als gestuft puffernder Kaskadenreaktor eingesetzt, lässt sich die Ausbeute beim Ernten von exprimierten Nutzkörpern massgeblich verbessern.

Beispiel 6:

Die **Anwendung** der amphoteren Copolymerisate zur mehrstufigen Pufferung des **Nach- und Aufzuchtmediums für Wassertiere.**

Die erfindungsgemässe Anwendung der amphoteren Copolymerisate mit pH = pI-Werten von beispielsweise 5,6 und 7,2 dient zur Pufferung von **Aufzuchtmedien fur Fischbrut, Jungfische, Krebse und Weichtiere,** indem sie in Filtermembranen aus Kunststoff-Gewebe einpolymerisiert werden. Diese Filter werden in geeigneter Weise in Pumpen eingesetzt und haben zur Aufgabe, das ökologische Milieu für die Nach- und Aufzucht in einem Gleichgewicht zu halten, bei dem das Erfordernis gesunder Entwicklungs- und Wachstumsbedingungen einerseits mit jenem nach einem wirksamen Schutz gegen Eutrophierung durch Algen und Pilze anderseits verbunden ist. Auf diese Weise lässt sich die Ausbeute im besonders empfindlichen Frühstadium der Leistungszucht steigern.

Beispiel 7:

**Die Aufnahme von biologischen Flüssigkeiten wie z.B. Urin oder Wundsekrete durch immobilisierte Puffercopolymerisate MARIPERL®. ("Auto buffered beads")**

Um Urin von Säuglingen oder von Patienten aufzunehmen, welche diesen nicht zurückhalten können, werden Windeln verwandt, die aus mehrfachen Zelluloselagen und einer Plastik-Deckschicht bestehen. Bei starker Urinausscheidung kann eine gewöhnliche Windel jedoch nicht alle abgegebene Körperflüssigkeit aufnehmen.

Wir haben mithilfe der erfindungsgemäss zu verwendenden synthetischen Trägerampholyten **isoelektrische Pufferperlen MARIPERL®** hergestellt. Es sind hydrophile Trocken-Granulate, welche mit den abgesonderten Körperflüssigkeiten quellen. Ihr Absorptionsvermögen wurde mit im Handel erhältlichen Harzen verglichen. Die Prüfung umfasste: Trockenes Sephadex G-200 (das auf einer Dextran-Matrix aufgebaut ist), Bio-Gel P-200 (das eine neutrale Polyacrylamid-Matrix als Grundlage hat) und Bio-Gel A (das Agarose enthält) einerseits, sowie die isoelektrischen Pufferperlen MARIPERL® mit folgender Zusammensetzung: 5% T, 5% C, enthaltend 50 mM pK 6,2 und 25 mM pK 4,6 Morpholino-Abkömmling, pI 6,2 anderseits. Die Messungen wurden bei Zimmertemperatur vorgenommen.

Wie Figur 5 zu entnehmen ist, erreichen alle Präparate ihre maximale Quellung innert 2 Stunden. Allerdings treten dabei erhebliche Unterschiede auf. Agarose hält nur dreimal, Bio-Gel P-200 zwölfmal, Sephadex G-200 fünfzehnmal das Trockengewicht an Flüssigkeit zurück, wohingegen **die erfindungsgemässen Microperlen 20 Mal ihr Eigengewicht an Körperflüssigkeit zurückhalten.**

Ueberdies haben sie im Vergleich zum besten bisherigen Absorbermaterial (Sephadex G-200) den beachtlichen Vorteil, dass sie bei Belastung mit dem vollen Körpergewicht nur 5 % der aufgenommenen Flüssigkeit wieder freigeben, gegenüber 50 % für Sephadex G-200 (das zusammen mit der Körperflüssigkeit eine schlammige Masse bildet).

Die erfindungsgemäss zu verwendenden selbstpuffernden Microperlen MARIPERL® lassen sich ebenfalls auf einen pI von 7,2 einstellen und nach Sterilisation in Wundverbände einarbeiten.

N.B. MARIPERL® ist eine eingetragene Marke (reg. ™) der Firma **MARIGEN S.A., RIEHEN.**

## Patentansprüche

1. Die Verwendung zur Pufferung von wässrigen Nährlösungen für Landpflanzen-, Wassertier- und Zellkulturen, sowie zur Absorbierung und Pufferung von Urin und Wundsekreten eines Mittels, das als aktive Komponente einen synthetischen Trägerampholyten mit positiv und negativ geladenen, in einer Matrix unbeweglich gemachten Gruppen aufweist, welche eines oder mehrere der Elemente Kohlenstoff, Schwefel, Phosphor, Bor oder Stickstoff enthalten.

2. Die Verwendung gemäss Anspruch 1 eines Mittels, dadurch gekennzeichnet, dass die in der Matrix unbeweglich gemachten Gruppen Carboxyl-, Schwefelsäure-, Borsäure-, Phosphorsäure-, Säureester- oder mehrwertige organische Säuregruppen einerseits und Aminogruppen anderseits sind und dass der synthetische Trägerampholyt in Form von porösen Kügelchen, bzw. in fasriger Form aufgetragen auf Glas, auf Dialysebeutel, auf Papier, auf jeden Typ von Plastikmaterial, sowie auf anorganische oder organische Oberflächen vorliegt.

3. Die Verwendung gemäss Anspruch 1 eines Mittels, dadurch gekennzeichnet, dass die Matrix aus einem Polymeren besteht.

4. Die Verwendung gemäss Anspruch 1 eines Mittels, dadurch gekennzeichnet, dass die Matrix aus einem Copolymerisat besteht, welches aus dem monomeren 2-Morpholinoethylacrylamid und der monomeren 4-Acrylamidobuttersäure mit Acrylamid und mit N,N'-Methylen-bis-acrylamid hergestellt wird und einen pI = pH-Wert von 5,6 hat.

5. Die Verwendung gemäss Anspruch 1 eines Mittels, dadurch gekennzeichnet, dass die Matrix aus einem Copolymerisat besteht, welches aus dem monomeren 2-Morpholinopropylacrylamid und der monomeren 4-Acrylamidobuttersäure mit Acrylamid und mit N-Acryloyl-Glycin oder mit 2-Acrylamido-2-Methylpropansulfonsäure hergestellt wird und einen pI = pH-Wert von 7,2 hat.

## Claims

1. The use of an agent for buffering nutrient solutions for terrestrial plants, aquatic animals and cell cultures, and for absorbing and buffering urine and wound secretions, which contains as the active component a synthetic carrier ampholyte with positively and negatively charged groups comprising one or several of the elements Carbon, Sulfur, Phosphorus, Boron or Nitrogen.

2. The use according to claim 1) of an agent which consists of groups immobilized in the matrix being on the one hand carboxyl, sulfuric acid, boric acid, phosphoric acid, acid ester or multi-valence organic acid groups and on the other hand amino groups and whereby the synthetic carrier ampholyte is present in the form of porous beads, of fibers coated on glass, dialysis bags, paper, plastic material and on inorganic or organic surfaces.

3. The use according to claim 1) of an agent, the matrix of which consists of a polymer.

4. The use according to claim 1) of an agent in which the matrix is made up of a copolymerisate obtained from the monomeric 2-morpholino-ethylacrylamide and the monomeric 4-acrylamidobutyric acid with acrylamide and with N,N'-methylene-bis-acrylamide and having an isoionic point pI = pH-Value of 5,6.

5. The use according to claim 1) of an agent in which the matrix is made up of a copolymerisate obtained from the monomeric 3-morpholino-propylacrylamide and the monomeric 4-acrylamidobutyric acid with acrylamide and with N-acryloyl-glycine or with 2 acrylamido-2-methylpropanesulfonic acid and having an isoionic point pI = pH-Value of 7,2.

## Revendications

1. L'utilisation d'un agent tamponnant pour solutions nutritives pour plantes terrestres, animaux aquatiques et cultures de cellules ainsi que pour l'absorption et le tamponnage d'urine et de sécrétions de plaies qui contient comme composé actif un support amphotérique synthétique avec des groupes chargés positivement et négativement comprenant un ou plusieurs éléments de carbone, soufre, phosphore, bore ou nitrogène.

2. L'utilisation selon revendication 1) d'un agent consistant en groupes chimiques immobilisés dans la matrice qui sont du carboxyle, de l'acide sulfuriqe, de l'acide phosphorique, des esters acides ou des acides organiques polyvalents d'un côté et divers groupes amino de l'autre et où le support ampholyte synthétique est fixé sur sphères poreuses ou sur fibres coatées sur verre, des sacs de dialyse, du papier, des matières plastiques ou des surfaces organiques et inorganiques.

3. L'utilisation selon revendication 1) d'un agent dont la matrice consiste en un polymère.

4. L'utilisation selon revendication 1) d'un agent dont la matrice consiste en un copolymérisat obtenu avec le 2-morpholino-ethyl-acrylamide monomérique, l'acide 4-acrylamidobutyrique, l'acrylamide et le N,N'-methylène-bis-acrylamide et qui produit une valeur pI (point isoionique) = pH de 5,6.

5. L'utilisation selon revendication 1) d'un agent dont la matrice consiste en un copolymerisat obtenu avec le 3-morpholino-propylacrylamide monomérique, l'acide 4-acrylamidobutyrique, l'acrylamide et le N-acryloyl-glycine ou l'acide 2-acrylamido-2-methylpropanesulfonique et qui produit une valeur pI (point isoionique) = pH de 7,2.

FIGUR 1

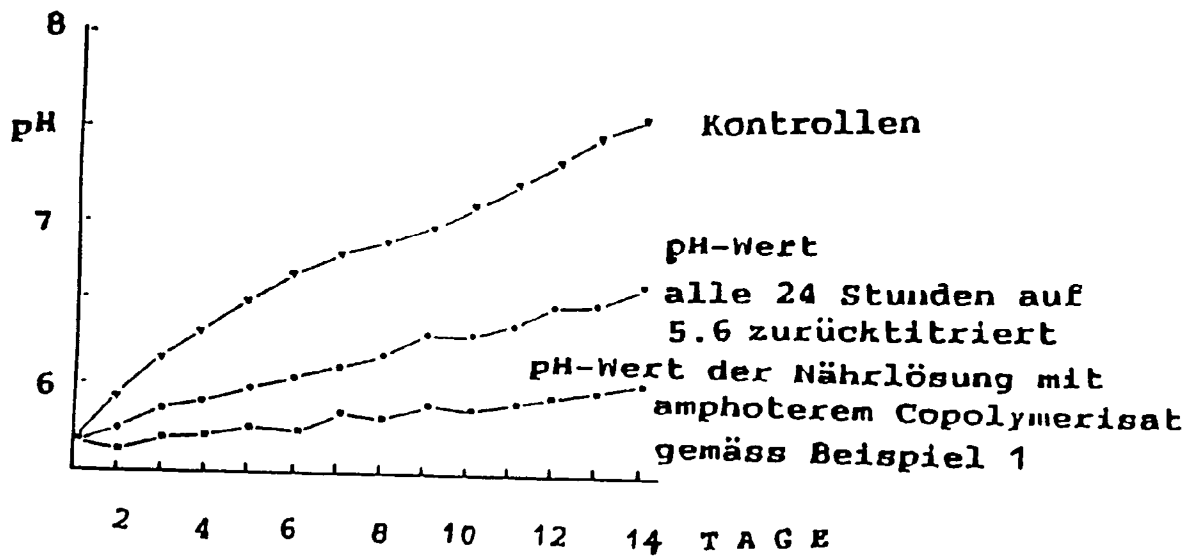


FIGUR 2

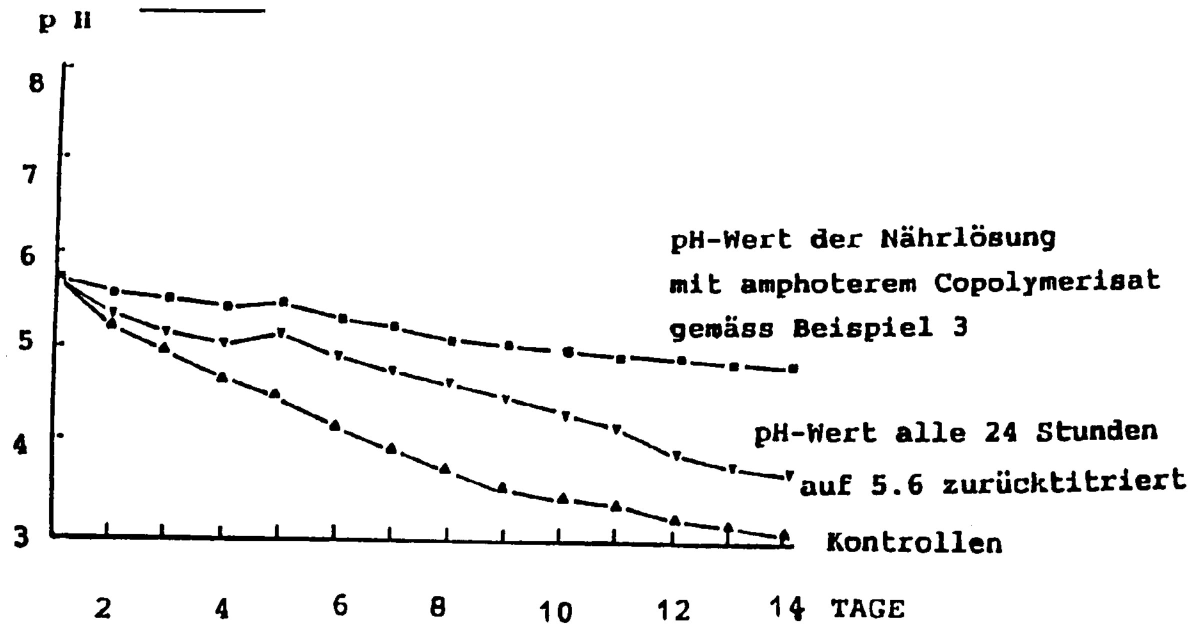

FIGUR 3

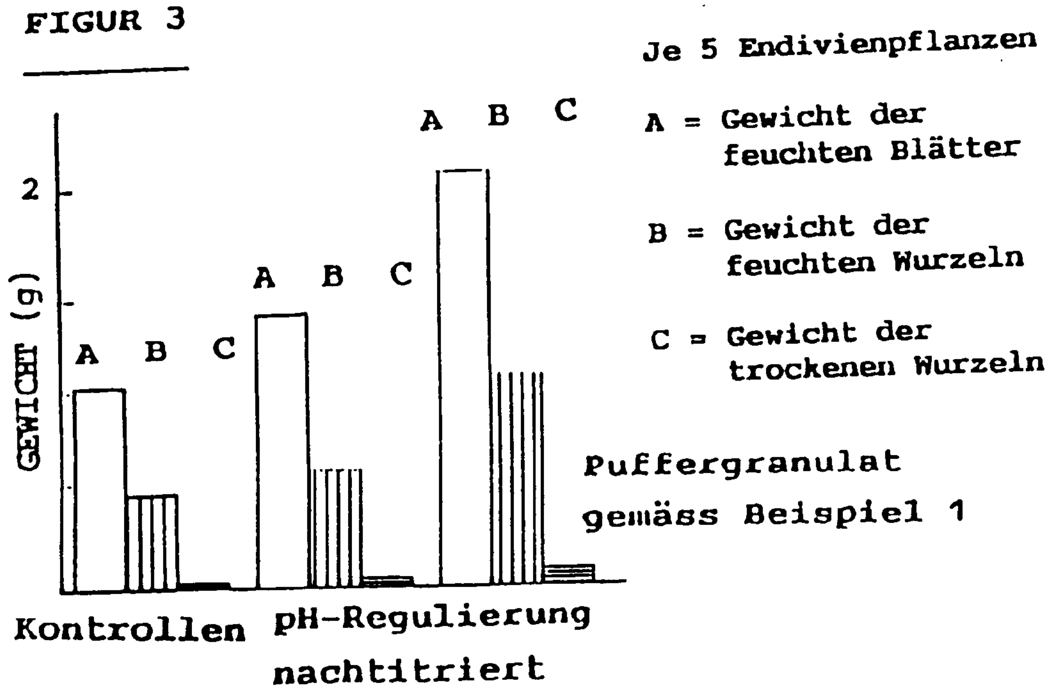


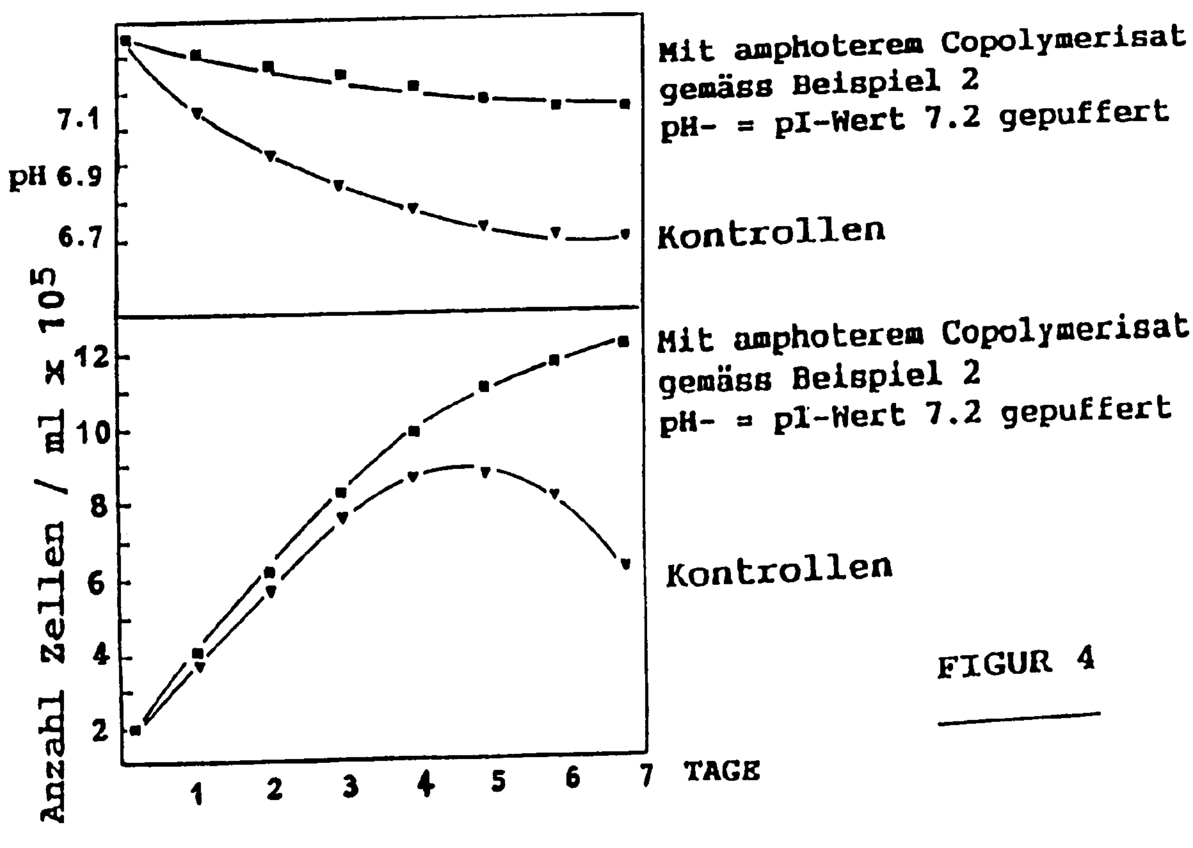


FIGUR 4

FIGUR 5

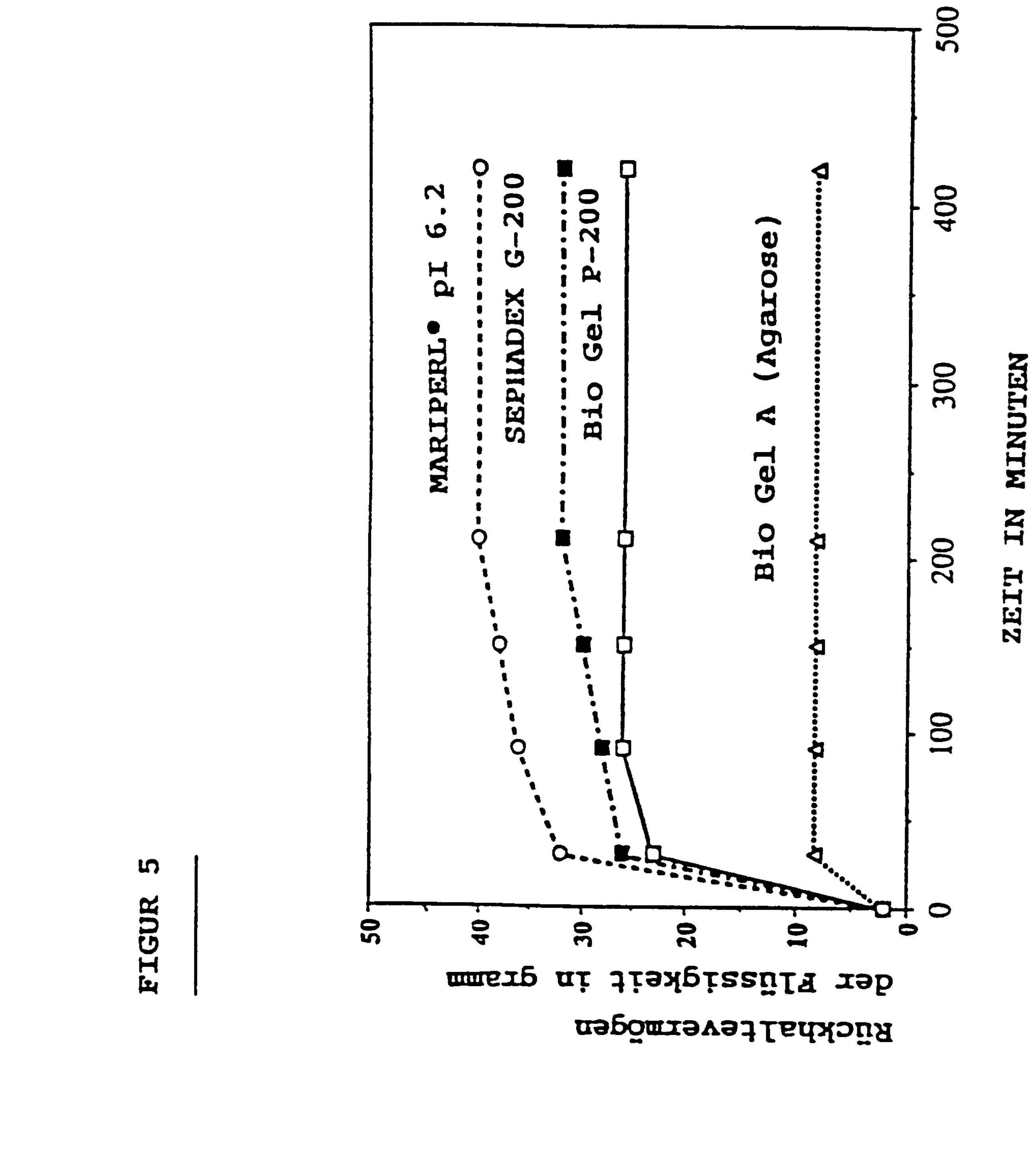
MARIPERL● pI 6.2
SEPHADEX G-200
Bio Gel P-200
Bio Gel A (Agarose)
Rückhaltevermögen
der Flüssigkeit in gramm
50
40
30
20
10
0
0
100
200
300
400
500
ZEIT IN MINUTEN